(19) Europäisches Patentamt
European Patent Office
Office européen des brevets

(11) Veröffentlichungsnummer: **0 134 972**
**B1**

(12) # EUROPÄISCHE PATENTSCHRIFT

(45) Veröffentlichungstag der Patentschrift: **20.11.86**

(51) Int. Cl.⁴: **C 07 C 63/40**, C 07 C 51/487, C 07 D 311/92

(21) Anmeldenummer: **84107830.6**

(22) Anmeldetag: **05.07.84**

(54) **Verfahren zur Herstellung von Naphthalin-1,4,5,8-tetracarbonsäure und deren 1,8-Monoanhydrid in hohem Reinheitsgrad.**

(30) Priorität: **09.07.83 DE 3324881**

(43) Veröffentlichungstag der Anmeldung: **27.03.85 Patentblatt 85/13**

(45) Bekanntmachung des Hinweises auf die Patenterteilung: **20.11.86 Patentblatt 86/47**

(84) Benannte Vertragsstaaten: **CH DE FR GB IT LI**

(56) Entgegenhaltungen:
**EP - A - 0 073 464**
**DE - A - 2 343 964**

**CHEMICAL ABSTRACTS, Band 98, nr. 20, 16. Mai 1983, Columbus, Ohio, USA, J. ZURAKOWSKA-ORSZAGH et al. "Polyimides from 1,4,5,8-naphtalenetetracarboxylic anhydride", Seite 5, Zusammenfassung Nr. 161 239y**

(73) Patentinhaber: **HOECHST AKTIENGESELLSCHAFT, Postfach 80 03 20, D-6230 Frankfurt am Main 80 (DE)**

(72) Erfinder: **Schiessler, Siegfried, Dr., Rother Weingartenweg 48, D-6232 Bad Soden am Taunus (DE)**
Erfinder: **Spietschka, Ernst, Dr., Kirchweg 3, D-6270 Idstein/Taunus (DE)**

EP 0 134 972 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Naphthalin-1,4,5,8-tetracarbonsäure (nachstehend als „NTC" bezeichnet) (Formel I) stellt ein wichtiges organisches Zwischenprodukt zur Herstellung von Farbstoffen und Pigmenten dar. Die NTC fällt bei der technischen Herstellung normalerweise in Form ihres 1,8-Monoanhydrids (Formel II) an, bei sehr hohen Trocknungstemperaturen auch als Dianhydrid (Formel III).

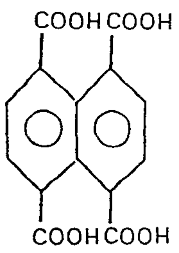
(I)

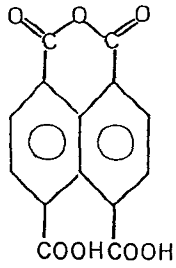
(II)

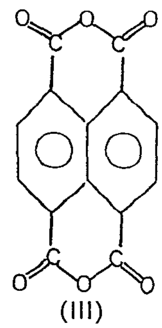
(III)

Das heute übliche Ausgangsprodukt für die Herstellung der NTC ist das bei der Destillation des Steinkohleteers anfallende technische Pyren. Das im Fiat Final Report 1313 II beschriebene Herstellungsverfahren für NTC, das von Acenaphthen ausgeht, hat keine wesentliche technische Bedeutung mehr. Die Herstellung der NTC, ausgehend von Pyren, kann bekanntermassen (vgl. z. B. Russian Chem. Rev. *34* (1965), S. 829-830) sowohl durch Direktoxydation als auch auf dem Umweg über 1,3,6,8-Tetrahalogenpyren erfolgen. Bei diesem zweiten Syntheseweg wird Pyren zunächst durch Chlorierung oder Bromierung in das 1,3,6,8-Tetrahalogenpyren überführt und dieses durch oxydative Enthalogenierung in Oleum zu 1,2,3,6,7,8-Hexahydro-1,3,6,8-pyrentetron bzw. zu dessen 2,7-Dibromderivat (im Fall des Tetrabrompyrens) umgesetzt. Aus diesen beiden Produkten kann sowohl durch saure als auch durch alkalische Oxydation die NTC erhalten werden.

Die nach den verschiedenen möglichen bekannten Syntheseverfahren erhaltene NTC ist in der Regel noch erheblich verunreinigt. Das ist zunächst darauf zurückzuführen, dass im Verlauf der Synthese durch Nebenreaktionen oder unvollständige Umsetzung Nebenprodukte entstehen. Ein Hauptgrund besteht aber auch darin, dass technisches Pyren nur etwa 90-95% Pyren enthält. Der Rest besteht aus polycyclischen Verbindungen von zum Teil bekannter (z. B. β-Brasan oder Fluoranthen) und zum Teil unbekannter Struktur, die im Rahmen der Herstellung von NTC ebenfalls Folgereaktionen ergeben, die zur Verunreinigung der NTC führen. Die Gewinnung von 100%igem Pyren kann wegen des sehr ähnlichen Dampfdrucks der polycyclischen Verunreinigungen auf destillativem Wege nicht auf wirtschaftliche Weise erfolgen. Eine Reinigung von Pyren durch Umkristallisation ist zwar möglich, bedeutet aber wegen der dabei auftretenden Pyrenverluste einen untragbaren wirtschaftlichen Aufwand.

Soweit die bei der Herstellung der NTC anfallenden Verunreinigungen in verdünnter Alkalilauge unlöslich sind, können sie relativ einfach dadurch abgetrennt werden, dass die verunreinigte NTC in wässeriger Alkalilauge in Form des Tetraalkalimetallsalzes gelöst wird und die unlöslichen Verunreinigungen durch Filtration abgetrennt werden. Das gleiche gilt selbstverständlich auch dann, wenn die NTC im Rahmen des Syntheseverfahrens (z. B. bei einer alkalischen Oxydation) in Form einer wässerigen Lösung ihres Tetraalkalimetallsalzes auftritt. Ein Teil der Verunreinigungen ist aber ebenfalls in wässerig-alkalischem Medium löslich und kann deshalb im Rahmen der Filtration nicht abgetrennt werden. Wird die NTC, wie im Fiat Final Report 1313 beschrieben, durch Ansäuern der wässerigen Lösung des Tetraalkalimetallsalzes der NTC ausgefällt, dann fallen erfahrungsgemäss auch die Verunreinigungen mit aus. Man erhält deshalb die NTC bzw. ihre Anhydride je nach der Synthesemethode mit einer Reinheit von nur 85-95%.

Für die Herstellung von Farbstoffen und Pigmenten auf Basis von NTC ist es aber ausserordentlich wichtig, dass eine NTC von sehr hoher Reinheit verwendet wird. Bei Einsatz von verunreinigter NTC wird erfahrungsgemäss eine Reihe von Farbstoff- bzw. Pigmenteigenschaften, wie z. B. die Reinheit des Farbtons, sehr negativ beeinflusst.

Es wurde gefunden, dass man Naphthalin-1,4,5,8-tetracarbonsäure (nachstehend als „NTC" bezeichnet) und deren 1,8-Monoanhydrid in hohem Reinheitsgrad (98-100%iger Reinheit) erhalten kann, indem man eine verunreinigte NTC oder ein verunreinigtes NTC-1,8-Monoanhydrid in bekannter Weise als Tetraalkalimetallsalz der NTC in Wasser löst, ggf. vorhandene unlösliche Verunreinigungen abtrennt, zweckmässigerweise durch Filtrieren, die geklärte wässerige Lösung unterhalb von 45° C auf einen pH-Wert von 4-5 ansäuert, das hierbei auskristallisierende schwerlösliche Dialkalimetallsalz der NTC abtrennt, zweckmässigerweise durch Filtrieren – die Verunreinigungen bleiben hierbei in Lösung – und anschliessend das Dialkalimetallsalz der NTC durch Behandeln mit einer starken Säure bei 20-100° C in die freie NTC bzw. das freie NTC-1,8-Monoanhydrid überführt.

Das Verfahren wird im einzelnen zweckmässigerweise so durchgeführt, dass die verunreinigte NTC bzw. das verunreinigte NTC-1,8-Monoanhydrid in der 10- bis 50fachen, vorzugsweise 20- bis 25fachen Menge Wasser in Gegenwart der mindestens äquivalenten Menge – bezogen auf die 4 Carbonsäuregruppen der NTC – eines Alkalicarbonats, wie beispielsweise Soda oder Pottasche, oder vorzugsweise eines Alkalihydroxids, wie Natrium- oder Kaliumhydroxid, als Tetraalkalimetallsalz gelöst wird. Falls sich hierbei keine klare Lösung ergibt, wird, ggf. in Gegenwart eines Klärhilfsmittels, geklärt (filtriert). Sofern im Zuge eines Syntheseverfahrens der NTC diese bereits in Form einer wässerigen Lösung ihres Tetraalkalimetallsalzes anfällt, kann diese Lösung in gleicher Weise als Ausgangslösung für das erfindungsgemässe Verfahren eingesetzt werden.

Die Lösung wird anschliessend bei einer Temperatur unterhalb von 45° C, vorzugsweise bei 0-30° C, durch langsames Zugeben einer mittelstarken bis starken Säure, vorzugsweise einer Mineralsäure, wie beispielsweise Salzsäure, Schwefelsäure oder o-Phosphorsäure, auf einen pH-Wert von 4-5 gestellt, wobei das Dialkalimetallsalz der NTC auskristallisiert. Durch Zugabe einer geringen Menge Alkalimetallsalz kann die Fällung vervollständigt werden. Das Dialkalimetallsalz der NTC wird abgetrennt, beispielsweise durch Filtration, und anschliessend durch Eintragen in eine starke Säure, d.h. eine Mineralsäure, wie beispielsweise Salzsäure, Schwefelsäure oder o-Phosphorsäure, in die NTC bzw. deren 1,8-Monoanhydrid überführt.

Unter Dialkalimetallsalzen der NTC werden hauptsächlich das Dikaliumsalz und das Dinatriumsalz verstanden, wobei das Dinatriumsalz von besonderem technischen Interesse ist.

Die Temperatur, bei der die Ausfällung des Dialkalimetallsalzes durch Ansäuern auf pH 4-5 erfolgt, muss unterhalb von 45° C liegen. Vorzugsweise erfolgt die Fällung bei einer Temperatur von 0-30° C. Temperaturen unterhalb von 0° C sind, soweit sie über dem Erstarrungspunkt der Mischung liegen, ebenfalls möglich. Man kann auch die Ausfällung des Dialkalimetallsalzes bei 20-30° C durchführen und erst anschliessend die Suspension zur Vervollständigung der Auskristallisation abkühlen. Die Ausfällung und Isolierung des Dialkalimetallsalzes der NTC bei Temperaturen zwischen 30° und unmittelbar unterhalb von 45° C ist zwar möglich, aber wenig zweckmässig, da bei einer Isolierung in diesem Temperaturbereich die Ausbeuteverluste wegen der Löslichkeit des Dialkalimetallsalzes zu hoch sind.

Wird die Ausfällung und Isolierung des Dialkalimetallsalzes der NTC bei 20-30° C durchgeführt, so ist es zweckmässig, durch Zugabe von ca. 5 Gew.-% (bezogen auf das Volumen der Suspension) an Alkalimetallchlorid die Fällung zu vervollständigen, während bei einer Isolierung des Dialkalimetallsalzes bei einer Temperatur von 0-10° C keine Zugabe von Alkalimetallchlorid erforderlich ist.

Beim Ansäuern einer Lösung des Tetraalkalimetallsalzes der NTC beginnt im pH-Bereich 5-6 das Dialkalimetallsalz der NTC auszukristallisieren. Zur Vollendung der Ausfällung wird auf den pH-Wert 4-5, vorzugsweise 4,5-4,8, angesäuert. Das Dialkalimetallsalz ist in der Suspension noch bis etwa pH 3 weitgehend beständig und wandelt sich bei niedrigeren pH-Werten in die NTC um. Zum Ansäuern auf pH 4-5 können an sich beliebige Säuren verwendet werden, doch werden Mineralsäuren, wie Salzsäure oder o-Phosphorsäure, bevorzugt.

Erfolgt die Einstellung des pH-Wertes 4-5 nicht bei Temperaturen unterhalb von 45° C, sondern im Temperaturbereich 45-100° C, so erhält man anstelle des Dialkalimetallsalzes der NTC mit steigender Temperatur in zunehmendem Masse das Dialkalimetallsalz des Monoanhydrids der NTC (Formel IV), das im Vergleich zum Dialkalimetallsalz der NTC eine wesentlich höhere Wasserlöslichkeit aufweist.

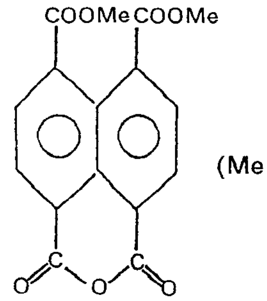

(IV)

(Me = Na oder K)

Beim Einstellen des pH-Wertes 4-5 im Temperaturbereich 80-100° C entsteht ausschliesslich das Dialkalimetallsalz des Monoanhydrids der Formel IV. Wird eine wässerige Suspension des Dialkalimetallsalzes der NTC einige Stunden gekocht, erfolgt ebenfalls weitgehend die Umwandlung in das Dialkalimetallsalz des Monoanhydrids der Formel IV. In gleicher Weise entsteht die Verbindung der Formel IV, wenn, wie aus Literaturbeispielen bekannt ist, das Monoanhydrid der NTC in Wasser in Gegenwart von zwei Äquivalenten Alkalilauge gelöst wird.

Während das Dialkalimetallsalz des Monoanhydrids der NTC (Formel IV) mit Ammoniak unter Bildung des Dialkalimetallsalzes des Monoimids der NTC reagiert, erfolgt beim Versuch der Umsetzung des Dialkalimetallsalzes der NTC mit Ammoniak keine derartige Reaktion. Weiterhin ist das aus der wässerigen Suspension isolierte Dialkalimetallsalz der NTC bei höherer Temperatur stabil und kann bei 100° C ohne Veränderung getrocknet werden.

Hinsichtlich der Struktur des Dialkalimetallsalzes der NTC soll keine Festlegung getroffen werden, doch erscheint die Struktur der nachstehenden Formel V

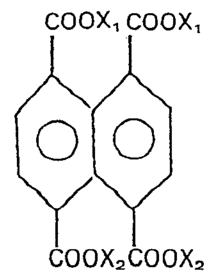

(V)

am wahrscheinlichsten, wobei ein $X_1$ und ein $X_2$ je ein Alkalimetallatom, das andere $X_1$ und $X_2$ je ein Wasserstoffatom bedeuten.

Die Dialkalimetallsalze der NTC zeichnen sich durch charakteristische Röntgenbeugungsspektren aus, die sich von den Spektren der Dialkalimetallsalze des Monoanhydrids der NTC wesentlich unterscheiden. Das Röntgenbeugungsspektrum des Dinatriumsalzes der NTC ist gekennzeichnet durch Reflexe bei Beugungswinkeln ($2\vartheta$, Cu-$K_\alpha$) von 9,50; 12,85; 14,50; 16,10; 17,25; 20,15; 26,40; 27,60; 28,05; 29,45 und 31,10°, wobei die intensiven Reflexe bei 17,25 und 20,15° besonders charakteristisch sind.

Das Röntgenbeugungsspektrum des Dikaliumsalzes der NTC ist gekennzeichnet durch Reflexe bei Beugungswinkeln ($2\theta$, Cu-$K_{\alpha}$) von 9,40; 10,90; 13,70; 16,30; 17,90; 19,55; 20,90; 23,35; 26,00; 27,10; 27,75; 29,75 und 30,55°.

Trägt man die Dialkalimetallsalze der NTC in eine verdünnte wässerige Säure, wie beispielsweise verdünnte Salzsäure, ein und behandelt einige Zeit bei 80-100° C, so erhält man das Monoanhydrid der NTC in so vorzüglich filtrierender Form, dass bei der Filtration ein Filterkuchen an Monoanhydrid mit einem Feststoffgehalt von 60-80% anfällt. Dieser Filterkuchen kann wegen des geringen Wassergehaltes ohne Trocknung direkt für die Herstellung von Farbstoffen und Pigmenten eingesetzt werden.

Man kann auch die Dialkalimetallsalze der NTC direkt für die Herstellung von Farbstoffen und Pigmenten einsetzen und dabei die NTC erst im Rahmen der Synthese freisetzen. So kann beispielsweise die Herstellung von ®Indanthren-Scharlach GG derart erfolgen, dass man das Dinatriumsalz der NTC mit o-Phenylendiamin in Äthanol in Gegenwart von zwei Äquivalenten einer starken Säure, wie Salz- oder Phosphorsäure, umsetzt. In gleicher Weise kann man durch Umsetzung des Dinatriumsalzes der NTC mit o-Phenylendiamin in siedendem Eisessig Indanthren-Scharlach GG kondensieren.

Die in den Beispielen genannten Teile und Prozente sind Gewichtsteile bzw. Gewichtsprozente.

*Beispiel 1:*

a) 143 Teile NTC-1,8-Monoanhydrid von 97%iger Reinheit werden bei 70-80° C in einer Lösung von 80 Teilen Ätznatron in 4000 Teilen Wasser gelöst. Dann wird bei 20-30° C in 40 Minuten durch Zutropfen von 124 Teilen 31%iger Salzsäure auf pH 4,8-4,5 gestellt, wobei das Dinatriumsalz der NTC in grobkristalliner Form ausfällt. Es wird 30 Minuten bei pH 4,8-4,5 nachgerührt. Dann werden 200 Teile Kochsalz zugesetzt. Dann wird 2-3 Stunden bei pH 4,8-4,5 nachgerührt, wobei der pH-Wert gegebenenfalls durch Zugabe einer geringen Menge Salzsäure nachgestellt wird. Anschliessend wird scharf abgesaugt und mit wenig Eiswasser nachgewaschen.

b) Der nach 1a) erhaltene Filterkuchen wird in eine Mischung von 1500 Teilen Wasser und 150 Teilen 31%iger Salzsäure bei 90-100° C eingetragen und 1 Stunde bei 90-100° C nachgerührt. Dann wird heiss abgesaugt, mit 1000 Teilen 1%iger Salzsäure gewaschen und bei 100° C im Vakuum getrocknet. Man erhält 136 Teile NTC-1,8-Monoanhydrid von 99-100%iger Reinheit.

c) Wird der nach Beispiel 1a) erhaltene Filterkuchen bei 100° C im Vakuum getrocknet, dann erhält man 166 Teile des Dinatriumsalzes der NTC. FIGUR 1 zeigt das Röntgenbeugungsspektrum (Cu-$K_{\alpha}$) dieser Verbindung. Die analytische Untersuchung ergibt folgenden Natriumwert:

berechnet (Mol 348): 13,2% Natrium
gefunden: 13,1% Natrium.

d) Zur Herstellung von Indanthren-Scharlach GG werden 104,4 Teile des nach Beispiel 1c) erhaltenen trockenen Dinatriumsalzes der NTC zusammen mit 70 Teilen 1,2-Diaminobenzol in eine Mischung von 500 Teilen Eisessig und 500 Teilen Wasser eingetragen und 6 Stunden unter gutem Rühren am Rückfluss gekocht. Dann wird heiss abgesaugt, mit heissem Wasser eisessigfrei gewaschen und bei 100° C im Vakuum getrocknet. Man erhält 121,7 Teile Indanthren-Scharlach GG.

*Beispiel 2:*

a) 143 Teile technisches NTC-1,8-Monoanhydrid von 91%iger Reinheit (hergestellt durch Salpetersäureoxydation von technischem 2,7-Dibrom-1,2,3,6,7,8-hexahydro-1,3,6,8-pyrentetron in Schwefelsäure) werden bei 70-80° C in einer Lösung von 80 Teilen Ätznatron in 4000 Teilen Wasser gelöst. Unlösliche Teile werden durch Filtration abgetrennt. Dann wird die Lösung in 2 Stunden bei 17-24° C durch Zutropfen von 468 Teilen 20%iger Orthophosphorsäure auf pH 4,8-5,0 gestellt, wobei das Dinatriumsalz der NTC auskristallisiert. Nach achtstündigem Nachrühren bei 20-25° C wird scharf abgesaugt. Die Weiterverarbeitung des Filterkuchens erfolgt analog Beispiel 1b). Man erhält 124 Teile NTC-1,8-Monoanhydrid von 99-100%iger Reinheit.

b) Stellt man die Lösung des Tetranatriumsalzes der NTC nicht bei 17-24° C, sondern bei 90-100° C auf pH 4,8-5,0, kühlt dann auf 20-25° C ab und rührt 8 Stunden bei 20-25° C nach, so kristallisiert ebenfalls Produkt aus, bei dem es sich im wesentlichen um das Dinatriumsalz des NTC-1,8-Monoanhydrids handelt. Wird das ausgefallene Produkt durch Filtration isoliert und analog Beispiel 2a) weiterverarbeitet, dann erhält man nur 85 Teile des NTC-1,8-Monoanhydrids.

*Beispiel 3:*

a) 143 Teile technisches NTC-1,8-Monoanhydrid von 92%iger Reinheit (hergestellt durch Oxydation von technischem ca. 90%igem 2,7-Dibrom-1,2,3,6,7,8-hexahydro-1,3,6,8-pyrentetron mit Chlorbleichlauge in verdünnter Natronlauge und anschliessendes Ausfällen der NTC mit Salzsäure) werden bei 70-80° C in einer Lösung von 80 Teilen Ätznatron in 3000 Teilen Wasser gelöst. Dann wird bei 20-30° C in 70 Minuten unter gutem Rühren durch Zutropfen von 124 Teilen 31%iger Salzsäure auf pH 4,8-4,5 gestellt, wobei das Dinatriumsalz der NTC auskristallisiert. Nach 1stündigem Nachrühren werden 100 Teile Kochsalz zugesetzt. Dann wird noch 8 Stunden bei 20-30° C und pH 4,8-4,5 gerührt. Schliesslich wird scharf abgesaugt. Die Weiterverarbeitung des Filterkuchens erfolgt analog Beispiel 1b). Man erhält 128,5 Teile NTC-1,8-Monoanhydrid von 99-100%iger Reinheit.

b) Verwendet man für das Auflösen des NTC-1,8-Monoanhydrids anstelle von 3000 Teilen nur 2000 Teile Wasser, so kann man vor der Isolierung des Dinatriumsalzes der NTC auf die Zugabe von Kochsalz verzichten. Man erhält in diesem Fall 129 Teile NTC-1,8-Monoanhydrid von 98,5-99%iger Reinheit.

*Beispiel 4:*

a) 4500 Teile einer wässerigen, schwach natronalkalischen, kochsalzhaltigen Lösung, die neben löslichen Verunreinigungen 196 Teile des Tetranatriumsalzes der NTC enthält (hergestellt durch Oxydation von technischem ca. 90%igem 2,7-Dibrom-1,2,3,6,7,8-hexahydro-1,3,6,8-pyrentetron in verdünnter Natronlauge mit Chlorbleichlauge), werden bei 20-30° C in 45 Minuten unter gutem Rühren durch Zutropfen von 161 Teilen 31%iger Salzsäure auf pH 5,0-4,5 gestellt, wobei das Dinatriumsalz der NTC in grobkristalliner Form auskristallisiert. Dann werden 150 Teile Kochsalz zugegeben und 2-3 Stunden bei 20-30° C unter pH-Kontrolle nachgerührt. Nach scharfem Absaugen wird der Filterkuchen in 1500 Teile Wasser eingetragen. Dann wird auf 90-100° C erhitzt. Anschliessend werden bei 90-100° C 200 Teile 31%iger Salzsäure zugetropft. Dann wird erneut 1 Stunde bei 90-100° C nachgerührt, heiss abgesaugt, mit 1000 Teilen 0,5%iger Salzsäure gewaschen und schliesslich bei 100° C im Vakuum getrocknet. Man erhält 139 Teile des NTC-1,8-Monoanhydrids von 98-99%iger Reinheit.

b) Wird die in Beispiel 4a verwendete Ausgangslösung bei 20-30° C direkt mit Salzsäure auf pH 1 angesäuert, dann 1 Stunde bei 90-100° C gerührt und analog Beispiel 4a isoliert, so erhält man ein NTC-1,8-Monoanhydrid von nur 91%iger Reinheit.

*Beispiel 5:*

143 Teile technisches NTC-1,8-Monoanhydrid von 92%iger Reinheit werden bei 70-80° C in einer Lösung von 80 Teilen Ätznatron in 3000 Teilen Wasser gelöst und die Lösung filtriert. Dann wird die Lösung auf 0-5° C abgekühlt. Darauf wird bei 0-5° C in 30 Minuten unter gutem Rühren durch Zutropfen von 126 Teilen 31%iger Salzsäure auf pH 4,5-5 gestellt, wobei das Dinatriumsalz der NTC auskristallisiert. Nach 1stündigem Nachrühren bei 0-5° C wird scharf abgesaugt, der Filterkuchen in 1500 Teile heisses Wasser eingetragen und bei 90-100° C mit 31%iger Salzsäure auf pH 1,5 gestellt. Dann wird erneut 1 Stunde bei 90-100° C nachgerührt. Schliesslich wird heiss abgesaugt, mit wenig Wasser salzfrei gewaschen und bei 100° C im Vakuum getrocknet. Man erhält 130 Teile NTC-1,8-Monoanhydrid von 99%iger Reinheit.

*Beispiel 6:*

a) 143 Teile NTC-1,8-Monoanhydrid von 92%iger Reinheit werden bei 70-80° C in einer Lösung von 130 Teilen 85%igem Kaliumhydroxid in 3000 Teilen Wasser gelöst. Dann wird bei 20-30° C in 40 Minuten durch langsame Zugabe von 130 Teilen 31%iger Salzsäure auf pH 4,8-4,5 gestellt. Anschliessend werden 100 Teile Kaliumchlorid zugegeben. Nach 3stündigem Nachrühren bei 20-30° C wird scharf abgesaugt.

b) Wird der nach Beispiel 6a) erhaltene Filterkuchen entsprechend Beispiel 1b) weiterverarbeitet, so erhält man 127 Teile NTC-1,8-Monoanhydrid von 98-99%iger Reinheit.

c) Wird der nach Beispiel 6a) erhaltene Filterkuchen mit wenig Eiswasser gewaschen und anschliessend bei 100° C getrocknet, so erhält man 166 Teile des Dikaliumsalzes der NTC. FIGUR 2 zeigt das Röntgenbeugungsspektrum (Cu-$K_\alpha$) dieser Verbindung.

## Patentansprüche

1. Verfahren zur Herstellung von Naphthalin-1,4,5,8-tetracarbonsäure (nachstehend als „NTC" bezeichnet) und deren 1,8-Monoanhydrid in hohem Reinheitsgrad, dadurch gekennzeichnet, dass man eine verunreinigte NTC oder ein verunreinigtes NTC-1,8-Monoanhydrid in bekannter Weise als Tetraalkalimetallsalz der NTC in Wasser löst, gegebenenfalls vorhandene unlösliche Verunreinigungen abtrennt, die geklärte Lösung unterhalb von 45° C auf den pH-Wert 4-5 stellt, das hierbei auskristallisierende Dialkalimetallsalz der NTC abtrennt und letzteres durch Behandeln mit einer starken Säure bei 20 bis 100° C in die freie NTC bzw. in das freie NTC-1,8-Monoanhydrid überführt.

2. Verfahren nach Anspruch 1, dadurch gekennzeichnet, dass man die Einstellung des pH der geklärten wässerigen Lösung des Tetraalkalimetallsalzes der NTC auf den Wert 4-5 mittels Salzsäure, Schwefelsäure oder o-Phosphorsäure vornimmt.

3. Verfahren nach Anspruch 1 oder 2, dadurch gekennzeichnet, dass man die Einstellung des pH-Wertes 4-5 bei einer Temperatur von 0° bis 30° C vornimmt.

4. Verfahren nach Ansprüchen 1-3, dadurch gekennzeichnet, dass man die geklärte wässerige Lösung des Tetraalkalimetallsalzes der NTC auf den pH-Wert 4,5-4,8 einstellt.

5. Verfahren nach Ansprüchen 1-4, dadurch gekennzeichnet, dass man die Überführung des abgetrennten Dialkalimetallsalzes der NTC in die freie NTC bzw. in das freie NTC-1,8-Monoanhydrid durch Eintragen des Dialkalimetallsalzes der NTC in Salzsäure, Schwefelsäure oder o-Phosphorsäure vornimmt.

6. Verfahren nach Ansprüchen 1-5, dadurch gekennzeichnet, dass man von einer wässerigen Lösung des Tetranatriumsalzes der NTC ausgeht, die im Zuge eines Syntheseverfahrens für die NTC anfällt.

7. Das Dinatriumsalz der NTC, gekennzeichnet durch ein Röntgenbeugungsspektrum mit Reflexen bei Beugungswinkeln ($2\theta$, Cu$K_\alpha$) von 9,50; 12,85; 14,50; 16,10; 17,25; 20,15; 26,40; 27,60; 28,05; 29,45 und 31,10°.

8. Das Dikaliumsalz der NTC, gekennzeichnet durch ein Röntgenbeugungsspektrum mit Reflexen bei Beugungswinkeln ($2\theta$, Cu$K_\alpha$) von 9,40;

10,90; 13,70; 16,30; 17,90; 19,55; 20,90; 23,35; 26,00; 27,10; 27,75; 29,75 und 30,55°.

## Claims

1. A process for the preparation of naphthalene-1,4,5,8-tetracarboxylic acid (called "NTC" below) and its 1,8-monoanhydride in a high degree of purity, which comprises dissolving impure NTC or impure NTC-1,8-monoanhydride as the tetra-alkali metal salt of NTC in water in known manner, removing any insoluble impurities present, bringing the clarified solution to a pH value of 4-5 below 45° C, separating off the di-alkali metal salt of NTC which thereby crystallizes out and converting the latter into free NTC or free NTC-1,8-monoanhydride by treatment with a strong acid at 20 to 100° C.

2. The process as claimed in claim 1, wherein the pH of the clarified aqueous solution of the tetra-alkali metal salt of NTC is brought to the value of 4-5 by means of hydrochloric acid, sulfuric acid or o-phosphoric acid.

3. The process as claimed in either of claims 1 and 2, wherein the pH value is brought to 4-5 at a temperature of 0° to 30° C.

4. The process as claimed in any one of claims 1 to 3, wherein the clarified aqueous solution of the tetra-alkali metal salt of NTC is brought to a pH value of 4.5-4.8.

5. The process as claimed in any one of claims 1 to 4, wherein the di-alkali metal salt of NTC which has been separated off is converted into free NTC or into free NTC-1,8-monoanhydride by introduction of the di-alkali metal salt of NTC into hydrochloric acid, sulfuric acid or o-phosphoric acid.

6. The process as claimed in any one of claims 1 to 5, wherein an aqueous solution of the tetrasodium salt of NTC which is obtained in the course of a synthesis process for NTC is used as the starting substance.

7. The disodium salt of NTC, which has an X-ray diffraction spectrum with reflexes at diffraction angles (2θ, Cu-$K_\alpha$) of 9.50; 12.85; 14.50; 16.10; 17.25; 20.15; 26.40; 27.60; 28.05; 29.45 and 31.10°.

8. The dipotassium salt of NTC, which has an X-ray diffraction spectrum with reflexes at diffraction angles (2θ, Cu-$K_\alpha$) of 9.40; 10.90; 13.70; 16.30; 17.90; 19.55; 20.90; 23.35; 26.00; 27.10; 27.75; 29.75 and 30.55°.

## Revendications

1. Procédé de préparation de l'acide naphtalène-tétracarboxylique-1,4,5,8 (nommé ci-dessous «NTC»), et de son mono-anhydride en 1,8, sous une forme très pure, procédé caractérisé en ce qu'on dissout dans de l'eau un NTC impur, ou un mono-anhydride en 1,8 du NTC impur, sous la forme d'un tétra-sel de métal alcalin du NTC, comme cela est connu, puis on sépare d'éventuelles impuretés insolubles, on règle le pH de la solution clarifiée à une valeur de 4 à 5, à une température inférieure à 45° C, on sépare le di-sel de métal alcalin du NTC qui a alors cristallisé et on transforme ce dernier, en le traitant par un acide fort, à une température de 20 à 100° C, en le NTC libre ou en le mono-anhydride en 1,8 du NTC libre.

2. Procédé selon la revendication 1, caractérisé en ce que, pour régler à une valeur de 4 à 5 le pH de la solution aqueuse clarifiée du tétra-sel de métal alcalin du NTC, on utilise de l'acide chlorhydrique, de l'acide sulfurique ou de l'acide orthophosphorique.

3. Procédé selon l'une des revendications 1 et 2, caractérisé en ce que le réglage du pH à 4-5 est effectué à une température de 0 à 30° C.

4. Procédé selon l'une quelconque des revendications 1 à 3, caractérisé en ce que le pH de la solution aqueuse clarifiée du tétra-sel de métal alcalin du NTC est réglé à une valeur de 4,5 à 4,8.

5. Procédé selon l'une quelconque des revendications 1 à 4, caractérisé en ce que la transformation du di-sel de métal alcalin du NTC, qui a été séparé, en le NTC libre ou en le mono-anhydride en 1,8 du NTC libre est effectuée par introduction du di-sel de métal alcalin du NTC dans de l'acide chlorhydrique, de l'acide sulfurique ou de l'acide orthophosphorique.

6. Procédé selon l'une quelconque des revendications 1 à 5, caractérisé en ce qu'on part d'une solution aqueuse du sel tétrasodique du NTC qui est obtenue au cours d'un procédé de synthèse du NTC.

7. Sel disodique du NTC, caractérisé par un spectre de diffraction des rayons X présentant des réflexions aux angles de diffraction (angle 2θ, raie $K_\alpha$ du cuivre) suivants: 9,50; 12,85; 14,50; 16,10; 17,25; 20,15; 26,40; 27,60; 28,05; 29,45 et 31,10°.

8. Sel dipotassique du NTC, caractérisé par un spectre de diffraction des rayons X présentant des réflexions aux angles de diffraction (angle 2θ, raie $K_\alpha$ du cuivre) suivants: 9,40; 10,90; 13,70; 16,30; 17,90; 19,55; 20,90; 23,35; 26,00; 27,10; 27,75; 29,75 et 30,55°.

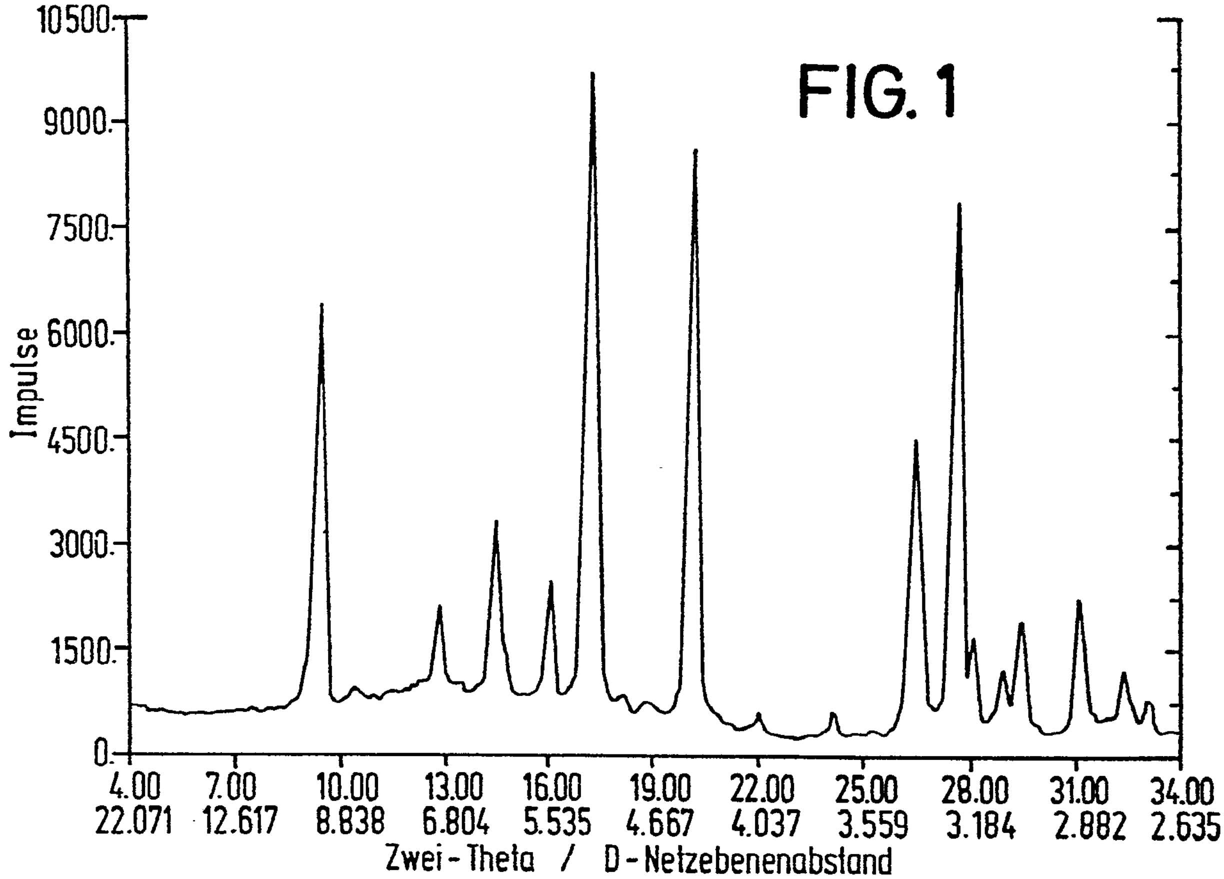
10500
9000
7500
6000
4500
3000
1500
0
Impulse
4.00 7.00 10.00 13.00 16.00 19.00 22.00 25.00 28.00 31.00 34.00
22.071 12.617 8.838 6.804 5.535 4.667 4.037 3.559 3.184 2.882 2.635
Zwei - Theta / D - Netzebenenabstand

FIG. 1

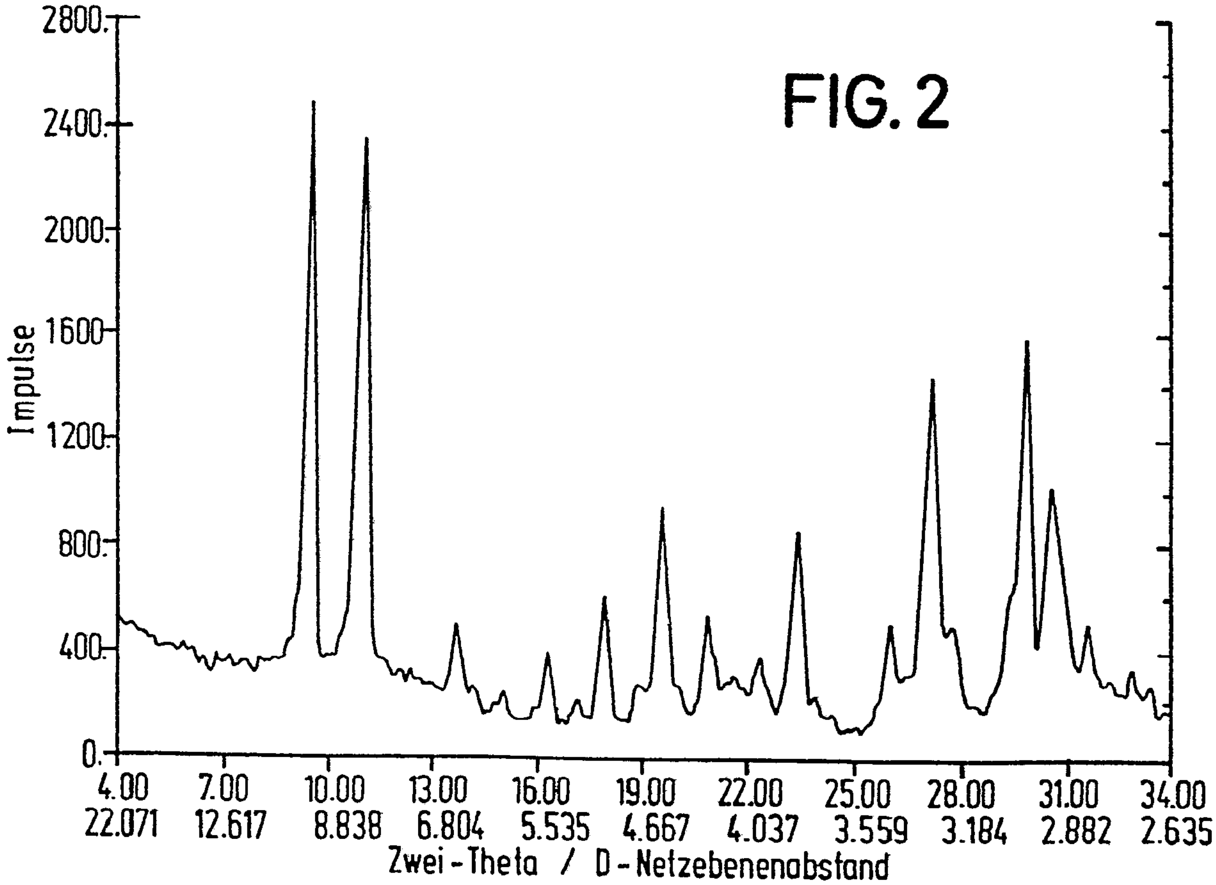
2800
2400
2000
1600
1200
800
400
0
Impulse
4.00 7.00 10.00 13.00 16.00 19.00 22.00 25.00 28.00 31.00 34.00
22.071 12.617 8.838 6.804 5.535 4.667 4.037 3.559 3.184 2.882 2.635
Zwei - Theta / D - Netzebenenabstand

FIG. 2